(19) Europäisches Patentamt
European Patent Office
Office européen des brevets

(11) Numéro de publication : **0 415 814 B1**

# (12) FASCICULE DE BREVET EUROPEEN

(45) Date de publication du fascicule du brevet : **01.07.92 Bulletin 92/27**

(51) Int. Cl.$^5$ : **B01J 27/08,** B01J 27/12, C07C 17/00, C07C 17/20

(21) Numéro de dépôt : **90402291.0**

(22) Date de dépôt : **13.08.90**

## (54) Nouveaux catalyseurs de fluoration en phase liquide.

(30) Priorité : **01.09.89 FR 8911490**

(43) Date de publication de la demande : **06.03.91 Bulletin 91/10**

(45) Mention de la délivrance du brevet : **01.07.92 Bulletin 92/27**

(84) Etats contractants désignés : **AT BE CH DE DK ES FR GB GR IT LI LU NL SE**

(56) Documents cités :
**EP-A- 0 301 346**
**DE-A- 1 041 026**
**US-A- 3 324 126**
**US-A- 4 374 289**
**US-A- 4 623 491**

(73) Titulaire : **ELF ATOCHEM S.A.**
**4 & 8, Cours Michelet La Défense 10**
**F-92800 Puteaux (FR)**

(72) Inventeur : **Laviron, Charles**
**27 A, Rue Soeur Bouvier**
**F-69005 Lyon (FR)**
Inventeur : **Lantz, André**
**Domaine de la Hêtraie**
**F-69390 Vernaison (FR)**

(74) Mandataire : **Leboulenger, Jean et al**
**ATOCHEM Département Propriété Industrielle**
**Cédex 42- La Défense 10**
**F-92091 Paris la Défense (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des catalyseurs constitués de mélanges d'halogénures d'antimoine trivalent et de titane tétravalent et leur utilisation pour la fluoration en phase liquide par l'acide fluorhydrique anhydre de dérivés aliphatiques halogénés, notamment chlorés.

La fluoration en phase liquide de dérivés chlorés aliphatiques, c'est-à-dire l'échange chlore-fluor avec de l'acide fluorhydrique anhydre, en phase liquide est une réaction très connue. Les chlorofluorocarbures les plus importants, c'est-à-dire $CFCl_3$, $CF_2Cl_2$, $CHF_2Cl$, $C_2Cl_3F_3$ peuvent ainsi être obtenus selon un tel procédé par échange chlore-fluor respectivement à partir de $CCl_4$, $CHCl_3$ et $C_2Cl_6$ (J.M. HAMILTON, "The Organic Fluorochemicals Industry" dans l'ouvrage Advances in Fluorine Chemistry, vol. 3, 1963, pages 146 à 150).

Les dérivés fluorés ou chlorofluorés aliphatiques à au moins deux atomes de carbone et contenant au moins un atome d'hydrogène peuvent être obtenus selon le même procédé de fluoration à partir des dérivés chlorés correspondants, mais ils peuvent, en général, aussi être otenus à partir d'oléfines chlorées par réaction avec de l'acide fluorhydrique selon une réaction dont le premier stade est une addition de HF à la double liaison. Ainsi, par exemple, $CF_3CH_3$ peut être obtenu, soit par fluoration de $CCl_3CH_3$, soit par fluoration de $CCl_2=CH_2$ (E.T. Mc BEE et al, I.E.C. 1947, pages 409 à 412). De la même façon, $CF_3CH_2Cl$ peut être obtenu par fluoration de $CCl_3CH_2Cl$ ou de $CCl_2=CHCl$ (A.K. BARBOUR et al, "The Preparation of Organic Fluorine Compounds by Halogen Exchange" dans l'ouvrage Advances in Fluorine Chemistry, vol. 3, 1963, pages 197-198). Est également décrite (EP-A-0301346) la préparation de polyfluorobutènes par fluoration de l'hexachlorobutadiène en présence de quantités catalytiques d'un halogénure de titane, trihalogénure d'antimoine et/ou pentahalogénure d'antimoine; les exemples de ce document utilisent $SbCl_5$, seul ou en mélange avec $SbF_3$.

Dans certains cas, avec des dérivés chlorés très réactifs, il est possible de réaliser ces fluorations par simple chauffage du dérivé chloré avec de l'acide fluorhydrique en absence de catalyseur. Il est ainsi connu que le méthylchloroformée $CCl_3CH_3$ peut être transformé en $CF_3CH_3$ par réaction avec de l'acide fluorhydrique anhydre, en phase liquide (E.T. Mc BEE et al, référence ci-dessus). Cette réactivité du méthylchloroforme est cependant tout-à-fait exceptionnelle et, en général, la réaction de l'HF en absence de catalyseur ne permet pas de réaliser l'échange chlore-fluor ou ne permet que de remplacer un seul atome de chlore et ceci même à des températures très élevées. Dans la pratique, les fluorations en phase liquide sont réalisées en présence d'un catalyseur de fluoration. Différents catalyseurs ont été proposés, mais les plus efficaces se sont révélés être les halogénures d'antimoine pentavalent ou les mélanges d'halogénures d'antimoine pentavalent et trivalent (HOUBEN-WEYL, Vol. V/3, 1962, page 126). Industriellement, on utilise en général le pentachlorure d'antimoine $SbCl_5$ ou un mélange de trichlorure d'antimoine $SbCl_3$ et de chlore qui fournissent, par action de l'acide fluorhydrique, des chlorofluorures mixtes, tels que $SbF_3Cl_2$ ou $SbF_2Cl_3$, qui se sont avérés être des catalyseurs de fluoration particulièrement efficaces. Les chlorofluorures d'antimoine pentavalent se décomposent cependant aux températures requises pour la fluoration et fournissent des halogénures d'antimoine trivalent et du chlore. Les halogénures d'antimoine trivalent étant pratiquement inefficaces en fluoration, les catalyseurs à base d'antimoine +5 perdent rapidement leur efficacité et leur activité ne peut être maintenue que si on arrive à maintenir l'antimoine à son degré d'oxydation +5. Ceci peut être réalisé par réoxydation par du chlore, c'est-à-dire en effectuant la fluoration en présence d'un peu de chlore qui permet de réoxyder en permanence $Sb^{+3}$ en $Sb^{+5}$ (référence HOUBEN-WEYL ci-dessus). Les catalyseurs à l'antimoine présentent néanmoins un certain nombre d'inconvénients :

– Les mélanges d'halogénures d'antimoine pentavalent et d'acide fluorhydrique sont très corrosifs, surtout à haute température.

– La fluoration en présence d'antimoine +5 est, dans certains cas, accompagnée de réactions parasites gênantes. Ainsi, dans le cas de dérivés chlorés contenant un atome d'hydrogène, il peut se former une oléfine par perte de HCl et ces oléfines peuvent donner lieu à la formation de produits lourds (HOUBEN-WEYL, référence ci-dessus, pages 134-135).

– La nécessité de réaliser la fluoration en présence de chlore peut aussi donner lieu à la formation d'un certain nombre de réactions secondaires. Ceci est en particulier le cas pour la fluoration d'hydrocarbures chlorés contenant encore un ou plusieurs atomes d'hydrogène, qui peuvent être remplacés par des atomes de chlore au cours de cette fluoration. Dans le cas où la matière première à fluorer est un dérivé éthylénique, il peut y avoir compétition entre une addition de HF ou une addition de chlore sur la double liaison. Dans le cas du trichloréthylène, on peut ainsi obtenir $CF_3CH_2Cl$ par addition de HF (HOUBEN-WEYL, page 107) ou $CF_2Cl\text{-}CFCl_2$ par chloration (HOUBEN-WEYL, page 134).

$$CCl_2{=}CHCl \xrightarrow{HF} CFCl_2{-}CH_2Cl \dashrightarrow CF_3CH_2Cl$$

$$CCl_2{=}CHCl \xrightarrow{Cl_2} CCl_3{-}CHCl_2 \xrightarrow[Cl_2/HF]{} CF_2Cl{-}CFCl_2$$

D'autres catalyseurs de fluoration en phase liquide ont été proposés. On peut par exemple citer les composés $SnCl_4$, $MoCl_5$, $WF_6$, $NbCl_5$, $TaF_5$, $TiCl_4$, $BF_3$, $CF_3SO_3H$, mais ces catalyseurs sont, en général, beaucoup moins efficaces que l'antimoine +5. Les halogénures de titane ont ainsi été proposés pour la préparation des chlorofluorométhane ou éthane par fluoration des dérivés chlorés correspondants (brevet US 2 439 299). Le tétrachlorure de titane peut aussi être utilisé pour la fluoration d'oléfines chlorées, telles que les tri ou tétrachloréthylènes (brevet US 4 374 289 et A.E. FEIRING, J. Fluor. Chem. 1979, 13, pages 7 à 18), par exemple :

$$C_2Cl_4 \rightarrow CFCl_2{-}CHCl_2 + CF_2Cl{-}CHCl_2$$
$$CCl_2{=}CHCl \rightarrow CFCl_2{-}CHCl_2$$

Cet halogénure de titane n'est cependant pas un catalyseur très puissant, car il ne permet en général que d'obtenir des produits monofluorés ou difluorés.

La demanderesse a découvert que, si les halogénures de $Ti^{+4}$ et de $Sb^{+3}$ sont des catalyseurs de fluoration en phase liquide peu efficaces, un mélange des deux fluorures de $Ti^{+4}$ et de $Sb^{+3}$ est par contre beaucoup plus efficace. Il permet par exemple de préparer $CF_3CH_2Cl$ à partir de $CCl_3CH_2Cl$ ou de $CCl_2{=}CHCl$ sans donner lieu aux mêmes inconvénients que les halogénures de $Sb^{+5}$ (nécessité d'utiliser du chlore pour réoxyder l'antimoine +3 en antimoine +5). D'autres exemples, non limitatifs, d'utilisation de ce catalyseur $TiF_4 | SbF_3$, sont la fluoration de dérivés chlorés du méthane comme $CCl_4$ , $CHCl_3$, $CH_2Cl_2$ ou de dérivés chlorés de l'éthane ou de l'éthylène comme $C_2Cl_6$ , $CCl_3CHCl_2$, $CCl_3CH_3$ $CCl_2{=}CCl_2$, $CCl_2{=}CH_2$, $CHCl_2CH_2Cl$ et $CHCl{=}CHCl$.

Bien que le catalyseur selon l'invention soit plus particulièrement destiné à fluorer les dérivés chlorés, il peut aussi être utilisé pour la fluoration de dérivés bromés ou iodés tels que, par exemple, $CBr_4$, $CHBr_3$, $CHBr_2Cl$, $CF_2Br_2$, $CBr_3CH_2Br$, $CHBr_2{-}CHBr_2$.

La forme active du catalyseur selon l'invention est un mélange de $TiF_4$ et de $SbF_3$. On peut ainsi engager un tel mélange de fluorures, mais on peut aussi utiliser un mélange des chlorures $TiCl_4$ et $SbCl_3$ , qui est transformé avec l'acide fluorhydrique en $TiF_4$ et $SbF_3$ avant ou pendant la fluoration de l'hydrocarbure halogéné à fluorer.

Les mélanges de $Ti^{+4}$ et $Sb^{+3}$ selon l'invention peuvent contenir 30 à 90 % molaires de $Ti^{+4}$ et 70 à 10 % molaires de $Sb^{+3}$. On préfère cependant utiliser, des mélanges où la proportion molaire de $Ti^{+4}$ est comprise entre 50 et 90 %.

La température de fluoration peut aussi varier dans de larges limites et on utilise, en général, des températures comprises entre 40°C et 180°C. La pression requise pour la réaction est au moins celle nécessaire pour maintenir le milieu réactionnel en phase liquide. Elle dépend donc essentiellement de la température réactionnelle et peut varier dans des limites aussi larges que 1 à 10 MPa (10 à 100 bars).

La réaction peut être réalisée en discontinu, dans un autoclave. Dans ce cas, on introduit dans l'autoclave le catalyseur (mélange $TiF_4$-$SbF_3$), ou le mélange $TiCl_4$-$SbCl_3$, l'acide fluorhydrique et le dérivé halogéné à fluorer et on porte le mélange, de préférence sous agitation, à la température de réaction. La pression de la réaction est dans ce cas la pression autogène.

La réaction peut aussi être réalisée en continu. La substitution d'un atome de chlore par un atome de fluor se traduisant par une diminution du point d'ébullition, il est par exemple possible d'introduire en continu, dans un réacteur contenant le catalyseur, l'acide fluorhydrique et le dérivé à fluorer et de soutirer en continu une phase gaz contenant l'acide chlorydrique formé, le dérivé fluoré et éventuellement de l'acide fluorhydrique. Dans ce cas, la réaction peut se dérouler à une pression constante qui doit être au moins égale à celle nécessaire pour maintenir le milieu réactionnel en phase liquide à la température de réaction.

La quantité de catalyseur peut varier dans de larges proportions. Le rapport molaire du catalyseur (antimoine plus titane) au réactif à fluorer peut ainsi varier de 0,1 à 0,6, mais des quantités plus ou moins importantes peuvent aussi être engagées. Cependant, pour les substrats difficiles à fluorer, on préfère en général utiliser des quantités correspondant à un rapport molaire de 0,2 à 0,4.

La proportion d'acide fluorhydrique à utiliser dépend, dans une large mesure, de la nature du dérivé halogéné à fluorer et peut varier dans de larges limites. On utilise néanmoins, en général, un excès d'HF, c'est-à-dire un rapport molaire HF/composé à fluorer supérieur à 1, ce rapport pouvant atteindre des valeurs très élevées, c'est-à-dire 5 à 20 ou même davantage.

Les exemples suivants illustrent l'invention sans la limiter. Ils ont été réalisés dans le matériel et selon la procédure décrits ci-après :

**Matériel** :

On a utilisé un autoclave de 800 ml, agité à l'aide d'un barreau magnétique et chauffé par l'intermédiaire d'huile circulant dans une double enveloppe. Il est équipé d'une prise de température, d'une mesure de pression et de deux piquages permettant de soutirer des produits, soit par la phase gaz, soit par la phase liquide.

**Procédure** :

Dans tous les cas, les réactifs, c'est-à-dire l'acide fluorhydrique et le composé à fluorer, sont introduits dans l'autoclave contenant le catalyseur et maintenu à la température de l'azote liquide.

Le réacteur est ensuite porté à la température de réaction et maintenu à cette température pendant un certain temps.

En fin de réaction, l'autoclave est refroidi jusqu'à la température ambiante. Il est ensuite décomprimé, puis purgé par un courant d'hélium à travers un laveur à eau pour éliminer les hydracides et un sécheur à $CaCl_2$. Les produits non absorbés sont piégés à la température de l'azote liquide. Le contenu du piège et éventuellement celui de l'autoclave sont ensuite analysés.

**EXEMPLE 1**

Dans l'autoclave décrit précédemment, on introduit 11,6 g de $SbCl_3$ (0,051 mole) et 9,7 g de $TiCl_4$ (0,051 mole). Après refroidissement à l'azote liquide, on introduit 87,3 g de $CCl_3CH_2Cl$ (0,52 mole) et 101,3 g de HF (5,06 mole).

L'autoclave est ensuite porté sous agitation à 150°C en deux heures et maintenu à cette température pendant 3 heures et demie. La pression atteint en fin de réaction 8,2 MPa (82 bars). Après refroidissement, les produits formés ont été récupérés selon la méthode décrite ci-dessus. On a ainsi obtenu 60,5 g de produit contenant 97 % de $CF_3CH_2Cl$ et 2 % de $CF_2ClCH_2Cl$, ce qui correspond à une conversion de $CCl_3CH_2Cl$ de 95 % en $CF_3CH_2Cl$ et de 2 % en $CF_2ClCH_2Cl$.

Dans l'autoclave, on recueille un solide légèrement hygroscopique, pesant environ 15,5 g et contenant essentiellement de l'antimoine, du titane et du fluor.

**EXEMPLE 2**

On introduit dans l'autoclave 5,7 g (0,025 mole) de $SbCl_3$ et 5 g de $TiCl_4$ (0,026 mole).

Après refroidissement, on introduit 20 g de HF, puis on porte l'autoclave à 50°C et on le maintient à cette température pendant une heure. Après refroidissement à la température ambiante, l'autoclave est dégazé et l'excès de HF et le HCl formé sont entièrement éliminés par un entraînement par un courant d'hélium. Il reste dans l'autoclave environ 7,5 g de produit correspondant pondéralement à un mélange de $TiF_4$ et de $SbF_3$.

On introduit ensuite à la température de l'azote liquide 40,3 g de $CCl_3CH_2Cl$ (0,24 mole) et 38,8 g de HF (1,94 mole).

L'autoclave est porté à 150°C en 2 heures et maintenu à 150°C pendant 3 heures et demie. La pression est montée en fin de réaction à 5,2 MPa (52 bars). On récupère selon la procédure habituelle 27,8 g de produits titrant 98,5 % de $CF_3CH_2Cl$ et 1 % de $CF_2ClCH_2Cl$.

**EXEMPLES 3, 4 ET 5**

Sans éliminer le catalyseur après le dégazage de l'essai 2, on recharge les réactifs trois fois de suite et on effectue ainsi 4 opérations successives sur la même charge de catalyseur (même température et même durée que pour l'exemple 2).

| n° essai | 3 | 4 | 5 |
|---|---|---|---|
| $CCl_3CH_2Cl$ (g) | 43,2 | 42,7 | 43,8 |
| HF (g) | 41,3 | 41,3 | 41,9 |
| Poids de produits obtenus (g) | 28,9 | 29 | 29,5 |
| Titre en $CF_3CH_2Cl$ | 99,6% | 99,5% | 99,1% |

Entre chaque essai, l'autoclave a été pesé et il n'y a eu aucune augmentation de poids. Après le dernier essais, l'autoclave a été ouvert et ne contenait, comme dans l'exemple 1, qu'un produit hygroscopique constitué uniquement d'antimoine, de titane et de fluor.

## EXEMPLE 6

Sur un catalyseur préparé, comme dans l'exemple 2, à partir de 5,7 g de $SbCl_3$, 5 g de $TiCl_4$ et de HF, on charge :

44,3 g de $CCl_3CH_2Cl$

41,6 g de HF

Le réacteur est porté en une heure et demie à 110°C et maintenu 4 heures à cette température. La pression est montée dans ce cas à 4,05 MPa (40,5 bars). L'analyse du produit récupéré (31 g) permet de calculer un taux de conversion du $CCl_3CH_2Cl$ de 71,8 % en $CF_3CH_2Cl$ et de 24,5 % en $CF_2ClCH_2Cl$ .

A titre de comparaison, ont été réalisés des essais sans catalyseur et avec des halogénures de titane et d'antimoine trivalent seuls.

## EXEMPLE COMPARATIF 7

On a chargé dans l'autoclave 24 g de $CCl_3CH_2Cl$ et 36 g de HF. Après 5 heures de réaction à 150°C, les produits récupérés ont permis d'établir le bilan suivant :

| | | |
|---|---|---|
| Taux de transformation $CCl_3CH_2Cl$ : | | 46 % |
| Conversion du $CCl_3CH_2Cl$ | en $CCl_2=CHCl$ | 17,6 % |
| | en $CFCl_2-CH_2Cl$ | 25,8 % |
| | en $CF_2Cl-CH_2Cl$ | 0,5 % |

## EXEMPLE COMPARATIF 8

On a préparé dans l'autoclave le fluorure de titane en faisant réagir, comme décrit dans l'exemple 2, 10,3 g de $TiCl_4$ (0,055 mole) et 20 g de HF (1 mole). On a ensuite introduit à froid 42 g de $CCl_3CH_2Cl$ (0,25 mole) et 52 g de HF (2,6 moles). Le mélange réactionnel a ensuite été porté en 2 heures à 150°C et maintenu à cette température pendant 3 heures et demie. Après dégazage, on a récupéré 26,5 g de produit contenant 54,8 % de $CF_3CH_2Cl$ et 38,4 % de $CF_2ClCH_2Cl$.

L'autoclave accuse dans ce cas une augmentation de poids de 1,7 g correspondant à 4 % du $CCl_3CH_2Cl$ engagé et constitué essentiellement de produits polymérisés.

## EXEMPLE COMPARATIF 9

On fait réagir comme précédemment 12 g de $SbCl_3$ (0,052 mole) et 20 g de HF (1 mole). On charge ensuite 43,7 g de $CCl_3CH_2Cl$ (0,26 mole) et 89 g de HF (4,45 mole). Après réaction dans les mêmes conditions que dans l'exemple 2, on a récupéré 32 g de produit dont l'analyse a permis de calculer les conversions du $CCl_3CH_2Cl$ :

3,3 % en $CF_3CH_2Cl$

79,4 % en $CF_2ClCH_2Cl$
4,2 % en $CFCl_2CH_2Cl$
3,2 % en $CCl_2$=CHCl

**EXEMPLE 10**

On prépare, comme décrit dans l'exemple 2, un catalyseur à partir de 11,4 g de $SbCl_3$ (0,05 mole), 9,4 g de $TiCl_4$ (0,05 mole) et 40 g de HF. Après élimination des acides HF et HCl, on a chargé 33,6 g de trichloréthylène (0,255 mole) et 80,7 g de HF (4,03 mole). Le mélange réactionnel a été porté en deux heures à 150°C et maintenu à cette température pendant 3 heures et demie. La pression est montée à 5,5 MPa (55 bars). Après dégazage, on a recueilli 29,5 g de produit contenant 97,9 % de $CF_3CH_2Cl$ et 2 % de $CF_2ClCH_2Cl$, ce qui correspond à une conversion du trichloréthylène en $CF_3CH_2Cl$ égale à 95,4 %.

**EXEMPLE 11**

On opère dans les mêmes conditions qu'à l'exemple 10, mais en engageant 4,8 g de $TiCl_4$ et 5,7 g de $SbCl_3$ et en chargeant 34,9 g de trichlorethylène et 40,6 g de HF. Après réaction à 150°C, la pression s'est stabilisée à 4,1 MPa (41 bars) et on a recueilli 31,5 g de produit contenant 10 % de $CF_3CH_2Cl$ et 89 % de $CF_2ClCH_2Cl$.

Après dégazage de l'autoclave, l'augmentation en poids de ce dernier correspondait à 6,9 g, c'est-à-dire un poids très voisin de celui du catalyseur.

**EXEMPLE 12**

Dans l'autoclave contenant le catalyseur de l'exemple 11, on a chargé 34,9 g de trichloréthylène (0,265 mole), 41,4 g de HF (2,07 mole) et 12,6 g de HCl (0,34 mole). La réaction a ensuite été réalisée à 150°C comme pour l'exemple 11 et on a atteint une pression de 6 MPa (60 bars). Après dégazage, on a recueilli 30,1 g de produit contenant 99,8 % de $CF_3CH_2Cl$, soit une conversion du trichloréthylène de 95,5 % en $CF_3CH_2Cl$.

**EXEMPLE 13**

Dans l'autoclave contenant le catalyseur utilisé pour l'exemple 10 et qui avait été obtenu à partir de 11,4 g de $SbCl_3$ et 9,4 g de $TiCl_4$, on a chargé 35 g de trichloréthylène et 82 g de HF. L'autoclave a ensuite été porté en une heure et demie à 110°C et maintenu à cette température pendant 4 heures. La pression s'est stabilisée à 2,9 MPa (29 bars). Après refroidissement et dégazage, on a recueilli 32,3 g de produit contenant 41,3 % de $CF_3CH_2Cl$ et 51,9 % de $CF_2ClCH_2Cl$.

**EXEMPLE 14**

Dans l'autoclave contenant le catalyseur de l'exemple 13, on a chargé 34,9 g de trichloréthylène et 81,6 g de HF. Le réacteur a été porté à 130°C en 2 heures et maintenu 3 heures et demie à cette température. On a récupéré après refroidissement et dégazage 30,5 g de produit contenant 76,7 % de $CF_3CH_2Cl$ et 16,4 % de $CF_2ClCH_2Cl$.

**EXEMPLE COMPARATIF 15**

En formant dans l'autoclave $SbF_3$ par réaction de 11,4 g de $SbCl_3$ et 20 g de HF et en chargeant ensuite 35 g de trichloréthylène et 82,3 g de HF, on a obtenu après réaction à 150°C pendant 5 heures et demie les résultats suivants :

| | |
|---|---|
| CCl2=CHCl non transformé | 17 % |
| Conversion en $CF_2ClCH_2Cl$ | 37,5 % |
| " " $CFCl_2CH_2Cl$ | 44,5 % |
| " " $CF_3CH_2Cl$ | 0,3 % |

En réalisant la réaction en présence uniquement de $TiF_4$ (obtenu à partir de 9,5 g de $TiCl_4$ et 20 g de HF) et avec 34,4 g de trichloréthylène et 40,7 g de HF, on a obtenu, après réaction à 150°C, une quantité très importante de polymères restant dans le réacteur et correspondant à plus que 40 % du trichloréthylène engagé. Les produits récupérés au cours du dégazage correspondaient par ailleurs à 14,6 g contenant 69 % de $CF_3CH_2Cl$ et 26 % de $CF_2ClCH_2Cl$.

**EXEMPLE 16**

Dans l'autoclave, on a chargé 11,6 g de $SbCl_3$ (0,05 mole) et 8,8 g de $TiCl_4$ (0,046 mole). Après refroidissement dans l'azote liquide, on a introduit 101,1 g de $CCl_3CHCl_2$ (0,5 mole) et 81,2 g de HF (4,06 mole). Le milieu a ensuite été porté en 2 heures à 150°C et maintenu à cette température pendant 3 heures et demie. Après refroidissement et dégazage, on a recueilli 81 g de produit contenant :

| | |
|---|---|
| $CF_2=CCl_2$ | 6,3 % |
| $C_2Cl_4$ | 4 % |
| $CF_3CHCl_2$ | 1 % |
| $CFCl_2CHCl_2$ | 3 % |
| $CF_2ClCHCl_2$ | 82 % |

**EXEMPLE 17**

On a opéré dans les mêmes conditions qu'à l'exemple 10, mais en engageant 4,75 g de $TiCl_4$ (0,025 mole) et 5,7 g de $SbCl_3$ (0,025 mole) et en chargeant, après la fluoration du catalyseur, 32,9 g de trichloroéthylène (0,25 mole) et 80 g de HF (4 moles). Le mélange réactionnel a été porté à 150°C en deux heures et maintenu à cette température pendant 3 heures et demie. La pression est montée à 5,2 MPa (52 bars). Après dégazage, on a recueilli 29 g de produit contenant 83 % de $CF_3CH_2Cl$, 14 % de $CF_2ClCH_2Cl$ et 2 % de $CFCl_2CH_2Cl$.

**EXEMPLE 18**

En opérant comme dans l'exemple 17, mais en engageant 3,57 g de $TiCl_4$ (0,0188 mole) et 7,12 g de $SbCl_3$ (0,031 mole), on a recueilli un produit contenant 55 % de $CF_3CH_2Cl$ et 44 % de $CF_2ClCH_2Cl$.

**EXEMPLE 19**

En opérant comme dans l'exemple 17, mais en engageant 7,12 g de $TiCl_4$ (0,0375 mole) et 2,85 g de $SbCl_3$ (0,0125 mole), on a recueilli un produit contenant 96 % de $CF_3CH_2Cl$ et 3 % de $CF_2ClCH_2Cl$.

**EXEMPLE 20**

En opérant comme dans l'exemple 17, mais en engageant 8 g de $TiCl_4$ (0,042 mole) et 1,8 g de $SbCl_3$ (0,0078 mole), on a obtenu un produit contenant 99,6 % de $CF_3CH_2Cl$.

**EXEMPLE 21**

Dans l'autoclave on a chargé 16 g de $TiCl_4$ (0,085 mole) et 3,6 g de $SbCl_3$ (0,016 mole), puis on a procédé à la fluoration du catalyseur avec 40 g de HF comme décrit dans l'exemple 2.

On a ensuite introduit 50,6 g de $CCl_3CHCl_2$ (0,25 mole) et 100 g de HF (5 moles). Le milieu réactionnel a été porté sous agitation à 150°C en deux heures et maintenu à cette température pendant trois heures et demie. La pression est montée et s'est stabilisée à 5,45 MPa (54,5 bars).

Après refroidissement et dégazage, on a recueilli 38 g de produit contenant 96,3 % de $CF_2Cl\text{-}CHCl_2$ et 2,3 % de $CF_3CHCl_2$.

## Revendications

1. Catalyseur de fluoration à base d'halogénures d'antimoine et de titane, caractérisé en ce qu'il est constitué d'un mélange de trifluorure d'antimoine et de tétrafluorure de titane, la proportion molaire de trifluorure d'antimoine étant comprise entre 10 et 70 % et celle de tétrafluorure de titane entre 90 et 30 %.

2. Catalyseur selon la revendication 1, dans lequel la proportion molaire de tétrafluorure de titane est comprise entre 50 et 90 %.

3. Catalyseur selon la revendication 1 ou 2, obtenu par fluoration d'un mélange de trichlorure d'antimoine et de tétrachlorure de titane.

4. Procédé de fluoration en phase liquide d'un hydrocarbure halogéné au moyen d'acide fluorhydrique, caractérisé en ce qu'on utilise un catalyseur selon l'une des revendications 1 à 3.

5. Procédé selon la revendication 4, caractérisé en ce que le catalyseur est formé par fluoration d'un

melange de trichlorure d'antimoine et de tétrachlorure de titane pendant la fluoration de l'hydrocarbure halogéné à fluorer.

6. Procédé selon la revendication 4 ou 5, dans lequel on opère à une température comprise entre 40 et 180°C.

7. Procédé selon l'une des revendications 4 à 6, dans lequel on opère en autoclave sous pression autogène.

8. Procédé selon l'une des revendications, 4 à 6, dans lequel on opère en continu à une pression comprise entre 1 et 10 MPa.

9. Procédé selon l'une des revendications 4 à 8, dans lequel le rapport molaire du catalyseur Sb+Ti au composé à fluorer est compris entre 0,1 et 0,6.

10. Procédé selon la revendication 9, dans lequel ledit rapport molaire est compris entre 0,2 et 0,4.

11. Procédé selon l'une des revendications 4 à 10, dans lequel le rapport molaire HF/composé à fluorer est supérieur à 1.

12. Procédé selon la revendication 11, dans lequel le rapport molaire HF/composé à fluorer est compris entre 5 et 20.

13. Procédé selon l'une des revendications 4 à 12, dans lequel l'hydrocarbure halogéné à fluorer est un dérivé chloré de l'éthane ou de l'éthylène.

## Patentansprüche

1. Katalysator zur Fluorierung auf der Basis von Antimon- und Titanhalogeniden, dadurch gekennzeichnet, daß er aus einem Gemisch aus Antimontrifluorid und Titantetrafluorid besteht, wobei der molare Anteil des Antimontrifluorids zwischen 10 und 70% und das des Titantetrafluorids zwischen 90 und 30% liegt.

2. Katalysator nach Anspruch 1, in dem der molare Anteil des Titantetrafluorids zwischen 50 und 90% liegt.

3. Katalysator nach Anspruch 1 oder 2, der durch die Fluorierung eines Gemisches aus Antimontrichlorid und Titantetrachlorid erhalten wurde.

4. Verfahren zur Fluorierung eines halogenierten Kohlenwasserstoffes in flüssiger Phase mit Hilfe von Fluorwasserstoffsäure, dadurch gekennzeichnet, daß man einen Katalysator nach einem der Ansprüche 1 bis 3 verwendet.

5. Verfahren nach Anspruch 4, dadurch gekennzeichnet, daß der Katalysator durch Fluorierung eines Gemisches aus Antimontrichlorid und Titantetrachlorid während der Fluorierung des zu fluorierenden halogenierten Kohlenwasserstoffes gebildet wird.

6. Verfahren nach Anspruch 4 oder 5, bei dem man bei einer Temperatur zwischen 40 und 180°C arbeitet.

7. Verfahren nach einem der Ansprüche 4 bis 6, bei dem man unter autogenem Druck im Autoklaven arbeitet.

8. Verfahren nach einem der Ansprüche 4 bis 6, bei dem man kontinuierlich bei einem Druck zwischen 1 und 10 MPa arbeitet.

9. Verfahren nach einem der Ansprüche 4 bis 8, bei dem das Molverhältnis des Sb+Ti-Katalysators zur zu fluorierenden Verbindung zwischen 0,1 und 0,6 liegt.

10. Verfahren nach Anspruch 9, bei dem das Molverhältnis zwischen 0,2 und 0,4 liegt.

11. Verfahren nach einem der Ansprüche 4 bis 10, bei dem das Molverhältnis HF/zu fluorierende Verbindung über 1 liegt.

12. Verfahren nach Anspruch 11, in dem das Molverhältnis HF/zu fluorierende Verbindung zwischen 5 und 20 liegt.

13. Verfahren nach einem der Ansprüche 4 bis 12, bei dem der zu fluorierende halogenierte Kohlenwasserstoff ein Chlorderivat von Ethan oder Ethylen ist.

## Claims

1. Fluorination catalyst based on antimony and titanium halides, characterised in that it comprises a mixture of antimony trifluoride and titanium tetrafluoride, the molar proportion of antimony trifluoride being between 10 and 70% and that of titanium tetrafluoride between 90 and 30%.

2. Catalyst according to Claim 1, in which the molar proportion of titanium tetrafluoride is between 50 and 90%.

3. Catalyst according to Claim 1 or 2, obtained by fluorination of a mixture of antimony trichloride and titanium tetrachloride.

4. Process for liquid phase fluorination of a halogenated hydrocarbon by means of hydrofluoric acid, characterised in that a catalyst according to one of Claims 1 to 3 is used.

5. Process according to Claim 4, characterised in that the catalyst is formed by fluorination of a mixture of antimony trichloride and titanium tetrachloride during the fluorination of the halogenated hydrocarbon to be fluorinated.

6. Process according to Claim 4 or 5, in which the reaction is carried out at a temperature of between 40 and 180°C.

7. Process according to one of Claims 4 to 6, in which the reaction is carried out in an autoclave under autogenous pressure.

8. Process according to one of Claims 4 to 6, in which the reaction is carried out continuously at a pressure of between 1 and 10 MPa.

9. Process according to one of Claims 4 to 8, in which the molar ratio of the Sb+Ti catalyst to the compound to be fluorinated is between 0.1 and 0.6.

10. Process according to Claim 9, in which the said molar ratio is between 0.2 and 0.4.

11. Process according to one of Claims 4 to 10, in which the molar ratio of HF/compound to be fluorinated is greater than 1.

12. Process according to Claim 11, in which the molar ratio of HF/compound to be fluorinated is between 5 and 20.

13. Process according to one of Claims 4 to 12, in which the halogenated hydrocarbon to be fluorinated is a chlorinated derivative of ethane or of ethylene.